# EUROPEAN PATENT APPLICATION

(11) **EP 3 240 262 A1**
(43) Date of publication of application: **01.11.2017**
(21) Application number: 15872999.6
(22) Date of filing: 21.12.2015
(51) Int. Cl.: H04M 1/00, A61B 5/00, A61B 5/0245, G06F 3/01

(54) **PORTABLE ELECTRONIC INSTRUMENT, CONTROL METHOD, AND CONTROL PROGRAM**

(30) Priority: 24.12.2014 JP 2014261289
(71) Applicant: Kyocera Corporation, Kyoto-shi, Kyoto 612-8501 (JP)
(72) Inventor: TANABE, Shigeki, Kyoto-shi Kyoto 612-8501 (JP); MORITA, Hideki, Kyoto-shi Kyoto 612-8501 (JP); MASUIKE, Isao, Kyoto-shi Kyoto 612-8501 (JP); SAITO, Shinya, Kyoto-shi Kyoto 612-8501 (JP)
(74) Representative: Schwabe - Sandmair - Marx
(86) International application number: PCT/JP2015/085682
(87) International publication number: WO 2016/104431

(57) **Abstract**

Conventional mobile electronic devices have a room for improvement in control on a plurality of sensors to detect information. For example, a mobile electronic device 1 includes a plurality of sensors, a detector that detects a biological reaction by one of the sensors, and at least one controller 10 that performs control to change a detection cycle of other sensors according to whether the biological reaction is detected. For example, the controller 10 changes the detection cycle used when the biological reaction is detected to a detection cycle shorter than the detection cycle used when no biological reaction is detected.

## Description

### Field

The present application relates to a mobile electronic device, a control method and a control program. Background

Mobile electronic devices with a function of acquiring and managing information of multiple sensors are known (for example, Patent Document 1).

### Citation List

### Patent Literature

Patent Document 1: Japanese Laid-open Patent Publication No. 2013-186539

### Summary

In the above-described mobile electronic device, there is room for improvement in control to detect information with a plurality of sensors.

According to one aspect, there is provided a mobile electronic device comprising: a plurality of sensors; a detector configured to detect a biological reaction by one of the sensors; and at least one controller configured to perform control to change a detection cycle of other sensors according to whether the biological reaction is detected.

According to one aspect, there is provided a control method that is executed by a mobile electronic device including a plurality of sensors, the control method comprising: detecting a biological reaction by one of the sensors; and performing control to change a detection cycle of other sensors according to whether the biological reaction is detected.

According to one aspect, there is provided a program for causing a mobile electronic device including a plurality of sensors to execute: detecting a biological reaction by one of the sensors; and performing control to change a detection cycle of other sensors according to whether the biological reaction is detected.

### Brief Description of Drawings

FIG. 1 is a diagram illustrating an example where a mobile device is worn.
FIG. 2 is a block diagram illustrating a functional configuration of the mobile device.
FIG. 3 is a flowchart illustrating an exemplary procedure of a sensor control process performed by the mobile device.
FIG. 4 is a flowchart illustrating another exemplary procedure of the sensor control process performed by the mobile device.

### Detailed Description

Multiple embodiments for an mobile electronic device, a control method, and a control program according to the present application will be described in detail below with reference to the drawings. A mobile device will be described below as an example of the mobile electronic device. Mobile devices include, for example, wearable devices, smartphones, feature phones, other mobile phones and handsets; however, the mobile devices are not limited thereto.

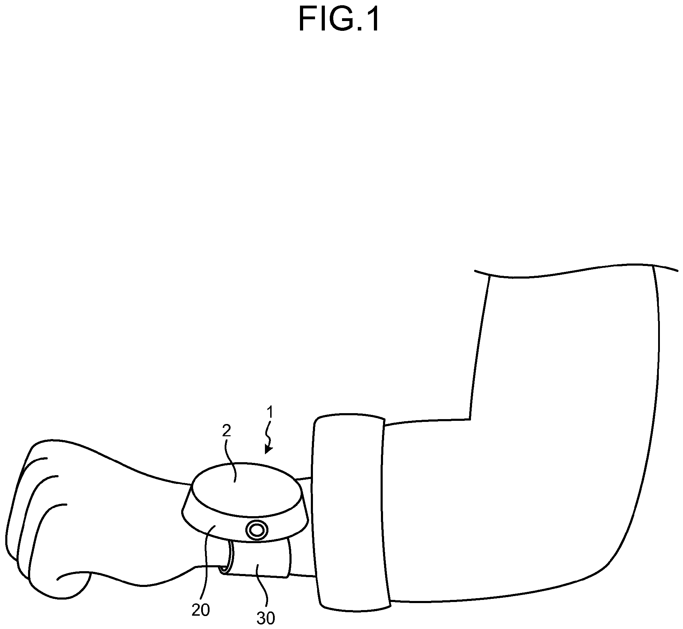

With reference to FIG. 1, an exemplary whole configuration of a mobile device 1 will be described. FIG. 1 is a diagram illustrating an example in which the mobile device 1 is worn. As illustrated in FIG. 1, the mobile device 1 is worn on the body of a user. In the example illustrated in FIG. 1, the mobile device 1 is worn on the wrist of the user. Wearing the mobile device 1 on the body of the user includes, for example, wearing the mobile device 1 on the wrist or the arm of the user or housing the mobile device 1 in a pocket of the user.

The mobile device 1 includes a body 20 and a wearing part 30. The body 20 is fixed to the wearing part 30. The wearing part 30 is a wrist band for wearing the body 20 on the body of the user. The mobile device 1 includes a state of being worn on the body of the user and a state of not being on the body of the user. The mobile device 1 is worn on the body of the user and accordingly collects information on the user. The information on the user includes, for example, biological information on the user, information on a travel state of the user and information on an environment around the user.

The mobile device 1 includes a touch screen display 2 that is provided to a main face (front face) of the body 20. The touch screen display 2 has a circular shape along a periphery of the main face. The touch screen display 2 has a function of displaying a screen containing various types of information including characters, diagrams and images. The touch screen display 2 further has a function of detecting a contact of various objects, such as a finger, a stylus and a pen. The mobile device 1 is able to determine user operations on the screen that is displayed on the touch screen display 2 based on the contact that is detected by the touch screen display 2.

The mobile device 1 includes multiple sensors. The mobile device 1 controls driving of multiple sensors and is able to detect various types of information with the sensors. The mobile device 1 is able to perform a process based on detection results by the sensors. The mobile device 1 is able to collect and save the detection results of the detection.

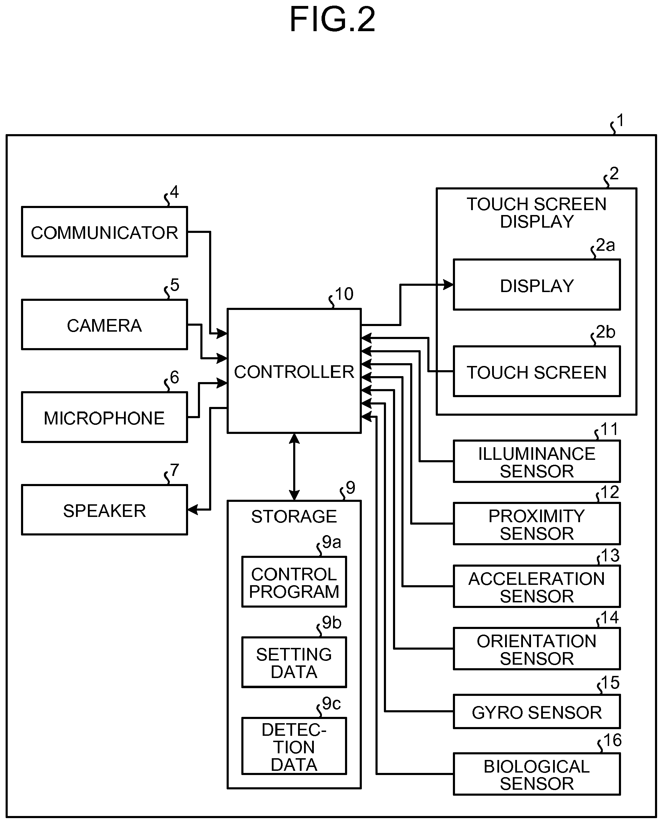

An exemplary functional configuration of the mobile device 1 will be described. FIG. 2 is a block diagram of the functional configuration of the mobile device 1. As illustrated in FIG. 2, the mobile device 1 includes the touch screen display 2, a communicator 4, a camera 5, a microphone 6, a speaker 7, a storage 9, a controller 10, an illuminance sensor 11, a proximity sensor 12, an acceleration sensor 13, an orientation sensor 14, a gyro sensor 15 and a biological sensor 16.

The touch screen display 2 includes a display 2a and a touch screen 2b that is superimposed onto the display 2a. The display 2a includes a display device, such as a liquid crystal display, an organic electro-luminescence display, or an inorganic electro-luminescence display. The display 2a is able to display characters, diagrams and images.

The touch screen 2b is able to detect the contact of a finger, a pen, or a stylus pen on the touch screen 2b. The touch screen 2b is able to detect a position in the touch screen 2b contacted by multiple fingers, a pen or a stylus pen.

The detection system of the touch screen 2b may be any method, for example, a capacitance method, a resistance film method, a surface acoustic wave method (or an ultrasound method), an infrared method, an electromagnetic induction method or a load sensing method. In order to simplify the following descriptions, it is assumed that the user contacts the touch screen 2b with the fingers in order to operate the mobile device 1.

The mobile device 1 is able to determine a type of gesture based on at least one of a contact that is detected by the touch screen 2b, a positon in which the contact is detected, a change of a position in which the contact is detected, a time interval between detected contacts and the number of times the contact is detected. The gesture includes operations performed on the touch screen 2b. The gesture that is determined by the mobile device 1 includes, for example, touch, long touch, release, swipe, tap, double tap, long tap, drag, flick, pinch-in and pinch-out; however, the gestures are not limited thereto.

The mobile device 1 is able to perform operations according to the gestures that are determined via the touch screen 2b. The mobile device 1 realizes intuitive and easy operability to users. The operations performed by the mobile device 1 according to the determined gestures include operations which may vary depending on the screen that is displayed on the display 2a. In order to simplify the following descriptions, "detection of the contact by the touch screen 2b, and determination, by the mobile device 1 based on the detected contact, that the type of a gesture is X" may be referred to as "detection of X by the smartphone" or "detection of X by the controller" below.

The communicator 4 communicates wirelessly. The communication system supported by the communicator 4 is a wireless communication standard. The wireless communication standard is, for example, a communication standard for, for example, 2G, 3G or 4G cellular phones. The cellular phone communication standard includes, for example, LTE (Long Term Evolution), W-CDMA (Wideband Code Division Multiple Access), CDMA2000, PDC (Personal Digital Cellular), GSM (trademark) (Global System for Mobile Communications) and PHS (Personal Handy-phone Systems). The wireless communication standard further includes, for example, WiMAX (Worldwide Interoperability for Microwave Access), IEEE802.11, Bluetooth (trademark), IrDA (Infrared Data Association) and NFC (Near Field Communication). The communicator 4 may support at least one of the communication standards listed above.

The camera 5 is able to convert captured images into electronic signals and output the electronic signals to the controller 10. The microphone 6 is able to convert a voice of a user into sound signals and output the sound signals to the controller 10. The speaker 7 is able to output sound signals that are transmitted from the controller 10 as sound.

The storage 9 is able to store a program and data. The storage 9 is also used as a work area in which processing results of the controller 10 are temporarily stored. The storage 9 may include any non-transitory storage medium, such as a semiconductor storage medium or a magnetic storage medium. The storage 9 may include multiple types of storage media. The storage 9 may include a combination of a portable storage medium, such as a memory card, an optical disk or a magneto-optical disk, and a storage-medium read device. The storage 9 may include a storage device that is used as a temporary storage area, such as a RAM (Random Access Memory).

The program that is stored in the storage 9 includes an application that is executed in the foreground or the background and a control program for implementing basic functions of the mobile device 1. The application, for example, is able to cause the display 2a to display a screen and cause the controller 10 to execute processes corresponding to the gestures that are detected via the touch screen 2b. The control program includes, for example, an OS. The application and the control program may be installed in the storage 9 via communications by the communicator 4 or a non-transitory storage medium.

The storage 9 is able to store, for example, a control program 9a, setting data 9b and detection data 9c. The control program 9a is able to provide functions on various types of control for causing the mobile device 1 to operate. The control program 9a is able to realize a call by, for example, controlling the communicator 4, the microphone 6 and the speaker 7. The functions that are provided by the control program 9a include a function of controlling the operations of the mobile device 1 according to the gestures on the touch screen 2b.

The setting data 9b is able to maintain various setting values on the operations of the mobile device 1. The setting data 9b contains, for example, determination conditions for determining that the mobile device 1 is worn on the body of the user. In the embodiment, the determination conditions contain conditions that are, for example, a determination threshold and a determination time for determining that the mobile device 1 is worn on the body of the user based on the detection result of the biological sensor 16.

It is possible to maintain information on the user that is detected by the sensors in the detection data 9c. The sensors include, for example, the illuminance sensor 11, the proximity sensor 12, the acceleration sensor 13, the orientation sensor 14, the gyro sensor 15 and the biological sensor 16; however, the sensors are not limited thereto. The sensors may include, for example, a temperature sensor, an ultraviolet sensor, and a GPS (Global Positioning System) receiver. In the detection data 9c, each of detection information representing the detection result by each of the sensors is stored. The detection information includes, for example, a time at which the sensor has performed the detection and values that are detected by the sensor.

The controller 10 includes an arithmetic processing unit. The arithmetic processing unit includes, for example, a CPU (Central Processing Unit), a SoC (System-on-a-chip), a MCU (Micro Controller), a FPGA (Field-Programmable Gate Array), and a co-processor; however the arithmetic processing unit is not limited thereto. The controller 10 may include multiple arithmetic processing units.

The controller 10 is able to control overall operations of the mobile device 1. The various functions of the controller 10 are implemented according to the control by the controller 10. Specifically, the controller 10 is able to refer to data that is stored in the storage 9 when required. The controller 10 controls various devices according to the data and commands. The controller 10 implements various functions by controlling the devices.

The controller 10 is able to control driving the multiple sensors by executing the control program 9a. The controller 10 is able to collect the results of the detection by the sensors and save the detection results in the detection data 9c. The controller 10 is able to perform a process corresponding to the detection results of the sensors.

The sensors are able to detect a subject to be detected at a detection cycle and output the detection results to the controller 10. Start and stop of the operations of the sensors are controlled by the controller 10. The sensors have a function of changing the detection cycle according to a change request from the controller 10. After changing the detection cycle in response to the change request from the controller 10, the sensors are able to detect the subject to be detected at the changed detection cycle.

In the exemplary embodiments, the detection cycle of each sensor is set in the setting data 9b. The setting data 9b contains cycle information. The cycle information has items such as a first detection cycle and a second detection cycle. The first detection cycle contains a cycle corresponding to a case where the mobile device 1 is worn on the body of the user. The first detection cycle may differ among all the sensors or part of the sensors. The first detection cycle may be common among all the sensors or part of the sensors. The second detection cycle contains a cycle corresponding to a case where the mobile device 1 is not worn on the body of the user. The second detection cycle may differ among all the sensors or part of the sensors. The second detection cycle may be common among all the sensors or part of the sensors. The first detection cycle of one sensor is set shorter than the second detection cycle. For some of the sensors, the same cycle may be set for both the case where the mobile device 1 is worn on the body of the user and the case where the mobile device 1 is not worn on the body of the user. For the second detection cycle, a cycle for reducing the power consumption of the mobile device 1 may be set. Setting the first detection cycle shorter than the second detection cycle makes it possible to increase the sampling rate of the sensor in the case where the mobile device 1 is worn on the body of the user higher than the sampling rate in the case where the mobile device 1 is not worn on the body of the user.

After executing the control program 9a to thereby detect a biological reaction based on the detection result by the biological sensor 16, the controller 10 is able to implement a process of changing the cycle of the detection by other sensors which are different from the biological sensor 16. Other sensors include all or part of the sensors which are different from the biological sensor 16.

The illuminance sensor 11 is able to detect illuminance of light surrounding the mobile device 1 at each detection cycle. The illuminance includes a value of a light flux incident on a unit area of a measurement surface of the illuminance sensor 11. The illuminance sensor 11 is, for example, used to adjust the luminance of the display 2a. The proximity sensor 12 is able to detect presence of an object in the vicinity without any contact. The proximity sensor 12 is able to detect the presence of the object based on, for example, a change in magnetic field or a change in a feedback time of a reflective wave of an ultrasound wave. The proximity sensor 12, for example, is able to detect that the touch screen display 2 is caused to get close to the face. The illuminance sensor 11 and the proximity sensor 12 may be configured as a single sensor. The illuminance sensor 11 may be used as a proximity sensor.

The acceleration sensor 13 is able to detect a direction and magnitude of acceleration act on the mobile device 1, an angle of inclination of the mobile device 1, and magnitude and a direction of acceleration of gravity at each detection cycle. The orientation sensor 14 is able to detect an orientation of the geomagnetism per detection cycle. The gyro sensor 15 is able to detect an angle and an angular velocity of the mobile device 1 at each detection cycle. The detection results of the acceleration sensor 13, the orientation sensor 14 and the gyro sensor 15 are used in combination to detect a change in the position, a change in an attitude and a state of the mobile device 1.

The biological sensor 16 is able to detect a biological reaction at each detection cycle. The biological sensor 16 may detect a heart rate as a biological reaction. The biological sensor 16 may detect electric signals emitted by the heart as the biological reaction. The biological sensor 16 may be configured by using, for example, an infrared sensor to detect the heart rate as the biological reaction. The biological sensor 16 may be configured by using, for example, an acceleration senor to detect the heart rate as the biological reaction. The biological sensor 16 may be configured by using, for example, a voltage sensor to detect electronic signals that are generated by the heart as the biological reaction.

The exemplary embodiments describe a case where the mobile device 1 detects the heart rate as the biological reaction by using the biological sensor 16; however, the embodiments are not limited thereto. For example, the mobile device 1 may be configured to detect the presence of the object in the vicinity as the biological reaction. For example, when the mobile device 1 is worn on the body of the user, an acceleration pattern which corresponds to movement of the user acts on the mobile device 1. In such a case, the mobile device 1 may be configured to detect the acceleration pattern corresponding to the movement of the user as the biological reaction.

Exemplary sensor control performed by the mobile device 1 according to the wearing state by the user illustrated in FIG. 1 will be described.

When the user does not wear the mobile device 1, the mobile device 1 causes the biological sensor 16 among the sensors to perform the detection at the first detection cycle and causes other sensors different from the biological sensor 16 to perform the detection at the second detection cycle longer than the first detection cycle. When the mobile device 1 is not worn on the body, other sensors perform the detection at a sampling rate lower than that used when the mobile device 1 is worn on the body. When the mobile device 1 is not worn on the body, the biological sensor 16 is not able to detect the biological reaction of the user. When the user does not wear the mobile device 1, the mobile device 1 collects the detection results of the detection by other sensors at the second detection cycle and saves the detection results in the detection data 9c.

Thereafter, when the mobile device 1 is worn on the body of the user as illustrated in FIG. 1, the biological sensor 16 detects the biological reaction of the user. When the mobile device 1 determines that the biological reaction is detected based on the detection result of the biological sensor 16, the mobile device 1 changes the detection cycle of other sensors to the first detection cycle shorter than the the second detection cycle. Accordingly, when the mobile device 1 is worn on the body, other sensors perform the detection at a sampling rate higher than that used when the mobile device 1 is not worn on the body. When the user wears the mobile device 1, the mobile device 1 collects the detection results of detection by other sensors at the first detection cycle and saves the detection results in the detection data 9c.

As described above, the mobile device 1 changes the cycle of the detection by other sensors depending on whether the biological reaction is detected. Accordingly, the mobile device 1 is able to perform autonomous control according to the wearing state by the user. When the biological reaction of the user is not detected, the mobile device 1 is able to reduce the power to be consumed when the mobile device 1 is not worn on the body of the user by changing the detection cycle of other sensors to a detection cycle longer than that used when the biological reaction is detected. As a result, lower power consumption and size reduction of a battery can be achieved to miniaturize the mobile device 1.

For example, power-saving of other sensors may be achieved by causing the user to perform on/off operations on a collecting function of the mobile device 1. Such a method, however, may bother the user and may lead a case in which data is not collected due to forgetting to perform the on operation on the collecting function by the user. In the mobile device 1 according to the present application, the user operations is not required by automatically changing the detection cycle of other sensors according to whether the mobile device 1 is worn on the body of the user. The mobile device 1 enables the power-saving without degrading the convenience.

Thereafter, when the user takes off the mobile device 1 from the body, the biological sensor 16 is not able to detect any biological reaction of the user. When the mobile device determines that the biological detection is not detected based on the detection result of the biological sensor 16, the mobile device 1 changes the detection cycle of other sensors to the second detection cycle longer than the first detection cycle. Accordingly, taking off the mobile device 1 from the body enables other sensors to perform the detection at a sampling rate lower than that used when the mobile device 1 is worn on the body. When the user does not wear the mobile device 1, the mobile device 1 is able to collect the detection results of detection by other sensors at the second cycle and saves the detection results in the detection data 9c.

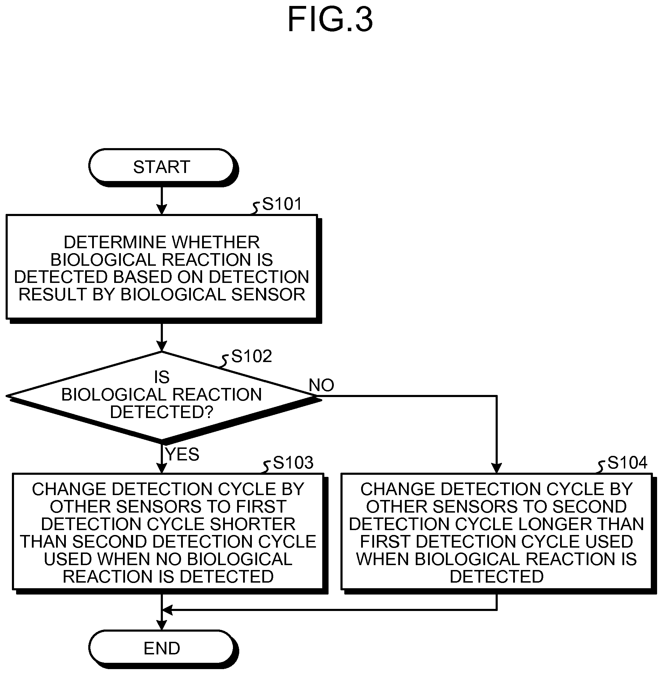

FIG. 3 is a flowchart illustrating an exemplary process procedure of sensor control performed by the mobile device 1. The process procedure illustrated in FIG. 3 is implemented by the controller 10 by executing the control program 9a. The process procedure illustrated in FIG. 3 is executed repeatedly.

As illustrated in FIG. 3, at Step S101, the controller 10 of the mobile device 1 determines whether the biological reaction is detected based on the detection result by the biological sensor 16. For example, when the biological reaction is detected by the biological sensor 16, the user highly likely wears the mobile device 1 on the body. For this reason, when the biological reaction is detected by the biological sensor 16, the controller 10 determines that the biological detection is detected. When the biological reaction is not detected by the biological sensor 16, the controller 10 determines that no biological detection is detected.

When the biological reaction is detected (YES at Step S102), the controller 10 proceeds to Step S103. At Step S103, the controller 10 changes the detection cycle of other sensors to the first detection cycle shorter than the second detection cycle used when no biological reaction is detected. Specifically, the controller 10 outputs a request for a change to the first detection cycle to each of other sensors different from the biological sensor 16. The controller 10 then ends the process procedure illustrated in FIG. 3.

When no biological response is detected (NO at Step S102), the controller 10 proceeds to Step S104. At step S104, the controller 10 changes the detection cycle of other sensors to the second detection cycle longer than the first detection cycle used when the biological reaction is detected. Specifically, the controller 10 outputs a request for a change to the second detection cycle to each of other sensors different from the biological sensor 16. Thereafter, the controller 10 ends the process procedure illustrated in FIG. 3.

Another example of the mobile device 1 will be described below. The mobile device 1 according to another example has the same configuration as that of the mobile device 1 illustrated in FIG. 2, except for the different function of the control program 9a.

Exemplary control of the mobile device 1 according to the wearing state by the user in another example will be described.

When the user wears the mobile device 1 on the body as illustrated in FIG. 1, the mobile device 1 causes other sensors different from the biological sensor 16 to perform the detection at the first detection cycle and causes the biological sensor 16 to perform the detection at a predetermined detection cycle. Thus, when the mobile device 1 is worn on the body, each of other sensors detects a subject to be detected. When the mobile device 1 is worn on the body, the biological sensor 16 detects the biological reaction of the user. When the user wears the mobile device 1, the mobile device 1 collects the detection results of the detection by other sensors at the first detection cycle and saves the detection results in the detection data 9c.

Thereafter, when the user takes off the mobile device 1 from the body, the biological sensor 16 is not able to detect any biological reaction of the user. When the mobile device determines that the biological reaction is not detected based on the detection result of the biological sensor 16, the mobile device 1 stops the detection by other sensors. For example, the mobile device 1 stops the detection by other sensors by changing the detection cycle of other sensors to a value which means stop of the operation. For example, the mobile device 1 stops the detection by other sensors by outputting a stop request to the multiple sensors. Accordingly, taking off the mobile device 1 from the body causes other sensors to stop the detection operation automatically. When the mobile device 1 is not worn on the body, the biological sensor 16 is not able to detect any biological reaction of the user. When the user does not wear the mobile device 1, the mobile device 1 does not collect the detection results of other sensors since other sensors are not in operation.

As described above, when the biological reaction is not detected by the biological sensor 16, the mobile device 1 according to the embodiment stops the detection by other sensors. Accordingly, the mobile device 1 is able to reduce the power consumed when the mobile device 1 is not worn on the body of the user according to the wearing state by the user.

Since the user operation is not required by controlling the start and the stop of the operations of other sensors according to whether the mobile device 1 is worn on the body of the user, the mobile device 1 enables the power-saving without degrading the convenience.

Thereafter, when the user wears the mobile device 1 on the body as illustrated in FIG. 1, the biological sensor 16 is able to detect the biological reaction of the user. When the mobile device 1 determines that the biological reaction is detected based on the detection result of the biological sensor 16, the mobile device 1 changes the cycle of the detection by other sensors to the first detection cycle from a stop state thereof. The mobile device 1 causes other sensors to start performing the detection at the first detection cycle. Accordingly, each of other sensors detects a subject to be detected when the mobile device 1 is worn on the body and do not perform the detection operation when the mobile device 1 is not worn on the body. When the user wears the mobile device 1, the mobile device 1 collects the detection results of the detection by other sensors at the first detection cycle and saves the detection results in the detection data 9c.

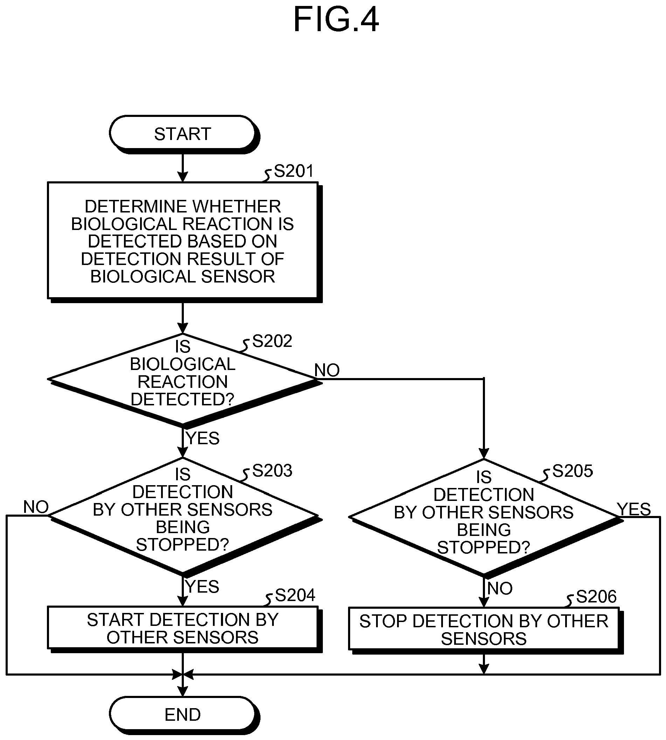

Another exemplary process procedure of the sensor control performed by the mobile device 1 will be described. FIG. 4 is a flowchart illustrating another exemplary process procedure of the sensor control performed by the mobile device 1. The process procedure illustrated in FIG. 4 is realized by the controller 10 by executing the control program 9a. The process procedure illustrated in FIG. 4 is executed repeatedly.

As illustrated in FIG. 4, at step S201, the controller 10 of the mobile device 1 determines whether the biological reaction is detected based on the detection result of the biological sensor 16. For example, when the biological sensor 16 detects the biological reaction, the user highly likely wears the mobile device 1 on the body. For this reason, the biological reaction is detected by the biological sensor 16, the controller 10 determines that the biological reaction is detected. When the biological reaction is not detected by the biological sensor 16, the controller 10 determines that no biological reaction is detected.

When the biological reaction is detected (YES at Step S202), the controller 10 proceeds to Step S203. The controller 10 determines whether the detection by other sensors is being stopped. When detection by other sensors is not being stopped (NO at Step S203), the controller 10 ends the process procedure illustrated in FIG. 4.

When the detection by other sensors is being stopped (YES at Step S203), the controller 10 proceeds to Step S204. The controller 10 starts the detection by other sensors. For example, the controller 10 causes the multiple sensors to perform the detection at the first detection cycle. Thereafter, the controller 10 ends the process procedure illustrated in FIG. 4.

When no biological reaction is detected (NO at Step S202), the controller 10 proceeds to Step S205. The controller 10 determines whether the detection by other sensors is being stopped as Step S205. When the detection by other sensors is being stopped (YES at Step S205), the controller 10 ends the process procedure illustrated in FIG. 4.

When the detection by other sensors is not being stopped (NO at Step S205), the controller 10 proceeds to Step S206. The controller 10 then stops the detection by other sensors at Step S206. Thereafter, the controller 10 ends the process procedure illustrated in FIG. 4.

The embodiments disclosed herein may be modified without departing from the scope of the present application. Furthermore, the embodiments disclosed herein may be combined as appropriate. For example, the above-described embodiments may be modified as described below.

For example, each program illustrated in FIG. 2 may be divided into multiple modules or may be associated with another program.

With respect to the above-described embodiments, the case where the mobile device 1 extends, shortens, or stops the cycle of the detection by other sensors different from the biological sensor 16 according to the wearing state by the user has been described; however the embodiments are not limited thereto. For example, the mobile device 1 may be configured to change the cycle of the detection by other sensors and the cycle of the detection by the biological sensor 16.

With respect to the above-described embodiments, the case where the mobile device 1 detects the biological reaction by the biological sensor has been described; however, the embodiments are not limited thereto. For example, the mobile device 1 may determine the biological reaction by a sensor different from the biological sensor 16 and change the cycle of the detection by other sensors including the biological sensor 16. For example, the mobile device 1 may change the detection cycle of other sensors (including the biological sensor 16) different from the proximity sensor 12 according to the result of the detection of an object by the proximity sensor 12. This enables the mobile device 1 to collect the results of the biological detection of the user by the biological sensor 16 according to the wearing by the user.

With respect to the above-described embodiments, the mobile device 1 has been described as an exemplary mobile electronic device; however, the mobile electronic device according to the appended claims is not limited to the mobile device 1. The mobile electronic device according to the appended claims may be a mobile electronic device other than the mobile device 1. The mobile electronic device includes, for example, a pedometer, a sleep meter, a tablet, a portable PC, a digital camera, a media player, an e-book reader, a navigator, and a game machine; however, embodiments are not limited thereto.

Descriptions of the characteristic embodiments have been given above in order to disclose the technology according to the appended claims completely and clearly; however, the appended claims should not be limited to the embodiments described above, and a configuration should be adopted so as to embody all modifications that can be created by those skilled in the art and alternative configurations within the scope of basic items illustrated herein.

In this application, description of "when", "during", "if", "in a case", "upon", "in response to determining", "in response to detecting" may be understood as another description depending on circumstances. In this application, description of "when 'a stated condition or event' is determined", "when 'a stated condition or event' is detected", or "upon determining 'a stated condition or even"', and description of "in response to determining", "upon detecting", or "in response to detecting" may be understood as another description depending on circumstances. In this application, description of "detect" may be understood as meaning of "measure", "scale", and "sense" depending on circumstances. In this application, description of "state" may be understood as "situation" depending on circumstances. For example, a moving state may be understood as a moving situation. A state of an electronic apparatus may be understood as a situation of an electronic apparatus.

### Reference Signs List

1
- 1: MOBILE DEVICE
- 2: TOUCH SCREEN DISPLAY
- 2a: DISPLAY
- 2b: TOUCH SCREEN
- 4: COMMUNICATOR
- 5: CAMERA
- 6: MICROPHONE
- 7: SPEAKER
- 9: STORAGE
- 9a: CONTROL PROGRAM
- 9b: SETTING DATA
- 9c: DETECTION DATA
- 10: CONTROLLER
- 11: ILLUMINANCE SENSOR
- 12: PROXIMITY SENSOR
- 13: ACCELERATION SENSOR
- 14: ORIENTATION SENSOR
- 15: GYRO SENSOR
- 16: BIOLOGICAL SENSOR

## Claims

1. A mobile electronic device comprising:
a plurality of sensors;
a detector configured to detect a biological reaction by one of the sensors; and
at least one controller configured to perform control to change a detection cycle of other sensors according to whether the biological reaction is detected.

2. The mobile electronic device according to claim 1, wherein the at least one controller is configured to change the detection cycle used when the biological reaction is detected to a detection cycle shorter than the detection cycle used when no biological reaction is detected.

3. The mobile electronic device according to claim 1 or 2, wherein the at least one controller is configured to change the detection cycle used when no biological reaction is detected to a cycle longer than the detection cycle used when the biological reaction is detected.

4. The mobile electronic device according to claim 1, wherein the at least one controller is configured to stop the detection by the other sensors when no biological reaction is detected.

5. A control method that is executed by a mobile electronic device including a plurality of sensors, the control method comprising:
detecting a biological reaction by one of the sensors; and
performing control to change a detection cycle of other sensors according to whether the biological reaction is detected.

6. A program for causing a mobile electronic device including a plurality of sensors to execute:
detecting a biological reaction by one of the sensors; and
performing control to change a detection cycle of other sensors according to whether the biological reaction is detected.
